# EUROPEAN PATENT SPECIFICATION

(11) **EP 1 820 730 B1**
(45) Date of publication and mention of the grant of the patent: **16.07.2008**
(21) Application number: 06121408.6
(22) Date of filing: 28.09.2006
(51) Int. Cl.: B65B 3/00, A61M 5/178, A61J 1/00

(54) **Dosing machine for radioactive liquid**
Dosiergerät für radioaktive Flüssigkeit
Machine de dosage pour liquide radioactif

(30) Priority: 21.02.2006 IT BO20060128
(43) Date of publication of application: 22.08.2007
(73) Proprietor: Tema Sinergie S.r.l., 48018 Faenza RA (IT)
(72) Inventor: PIANCASTELLI, Stefano, 48014, CASTEL BOLOGNESE (RA) (IT)
(74) Representative: Lanzoni, Luciano

(56) References cited:
- WO-A-96/31392
- WO-A-20/05002971
- WO-A-20/05118031
- US-A- 5 911 252
- US-A1- 2003 004 463
- US-A1- 2005 278 066

## Description

The present invention relates to a dosing machine for a radioactive substance and in particular to a dosing machine for radioactive liquids for medical or hospital use.

In the hospital sector frequent use is made of substances which are radioactive and which must therefore be handled appropriately.

For correct use of radioactive substances they must be precisely dosed and calibrated so that effective use can be made of them and the machines used for this purpose must have suitable safety standards.

Therefore, dosing machines are normally widely used both for filling shielded syringes with doses of radioactive substance and for filling vials; the radioactive substance used is normally supplied to the dosing machine from a synthesis module, if available, or from previously filled vials.

In document US 2005/278066 is disclosed an automated dispensing system for radioactive liquid. This system comprises at least three containers operatively connected for displacing radioactive liquid. Control valves and a processor regulate the amount of radioactive and of non-radioactive liquid that is selectively displaced, in order to limit the exposure of pharmacist personnel to the radioactive materials during handling.

In order to comply with standards of precision at every step of the transfer of the radioactive liquid, dosing machines of the known type are relatively complex and expensive, because they normally require the use of at least one pair of dose calibrators which are the substantially known instruments used to measure the activity (amount of radioactivity).

Moreover, known machines require laborious cleaning and disinfecting operations between one use and the next in particular for the successive treatment of separate patients or different substances.

Machines are also known which operate in the so-called "empty tube" mode. That is to say, after filling the individual syringe or vial, the tubes used for radioactive substance transit are completely emptied and the end of cycle residues or end of treatment waste are recovered by means of suitable tubing and pumps and stored in suitable containers if necessary.

In this context, the main technical purpose of the present invention is to propose a dosing machine which is free of the above-mentioned disadvantages. One aim of the present invention is to propose a dosing machine for radioactive substances which allows recovery of the end of cycle residues or the end of treatment waste without the use of dedicated tubing and pumps.

Another aim of the present invention is to propose a machine which allows the drug to be fractionated to be loaded and if necessary fractionated into vials without the use of dedicated pumps.

Yet another aim of the present invention is therefore to propose a dosing machine which is precise, relatively economical and whose filling process guarantees the sterility of the injectable product.

A further aim of the present invention is to propose a dosing machine which is practical to use for filling, in particular, vials (multi-dose or single-dose) and single-dose syringes.

Another aim of the present invention is the production of a dosing machine which is versatile and easy to use, in particular with a plurality of separate patients or different substances.

The stated technical purpose and aims are substantially fulfilled by a dosing machine with the technical characteristics described in one or more of the claims herein.

Further features and advantages of the present invention are more apparent in the description below, with reference to a preferred, non-limiting, embodiment of the invention, illustrated in the accompanying drawings, in which:
- Figure 1 is a schematic perspective view, partly in blocks, of a dosing machine in accordance with the present invention;
- Figure 2 is a schematic front view of the machine of Figure 1;
- Figure 3 is a schematic view of the cross-section III - III of the machine of Figure 2;
- Figure 4 is a diagram of the machine of Figure 1 in a first operating configuration;
- Figure 5 is a diagram of the machine of Figure 1 in a second operating configuration;
- Figure 6 is a diagram of the machine of Figure 1 in a third operating configuration;
- Figure 7 is a diagram of the machine of Figure 1 in a fourth operating configuration;
- Figures 8 to 10 illustrate three kits of disposable components which can be associated with the machine to allow various methods of use;
- Figures 11 to 16 illustrate different configurations of the kits of Figures 8 to 10, depending on corresponding methods of machine use.

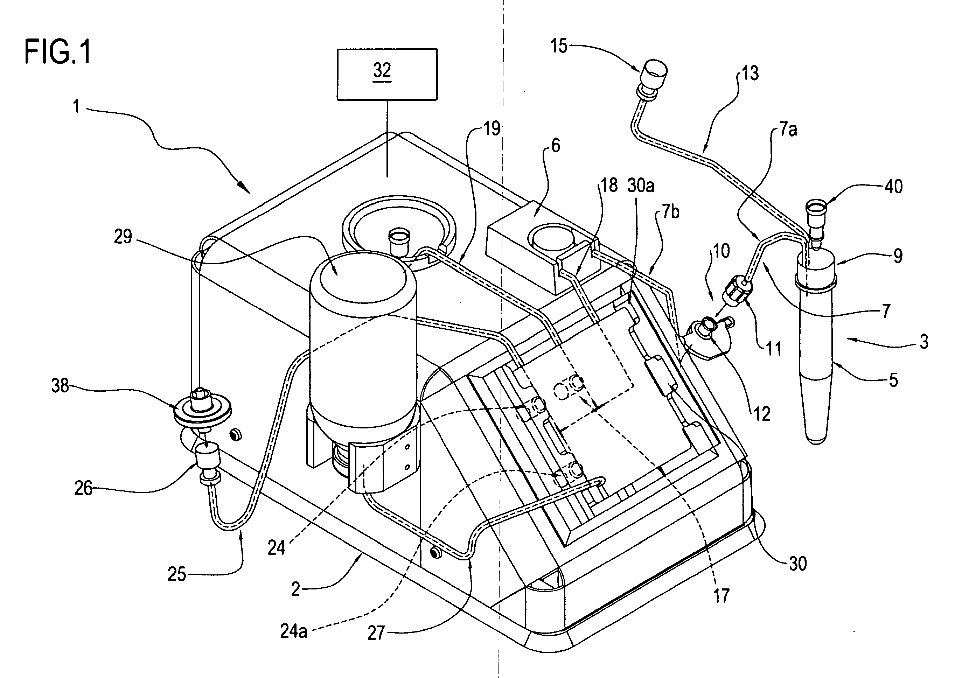

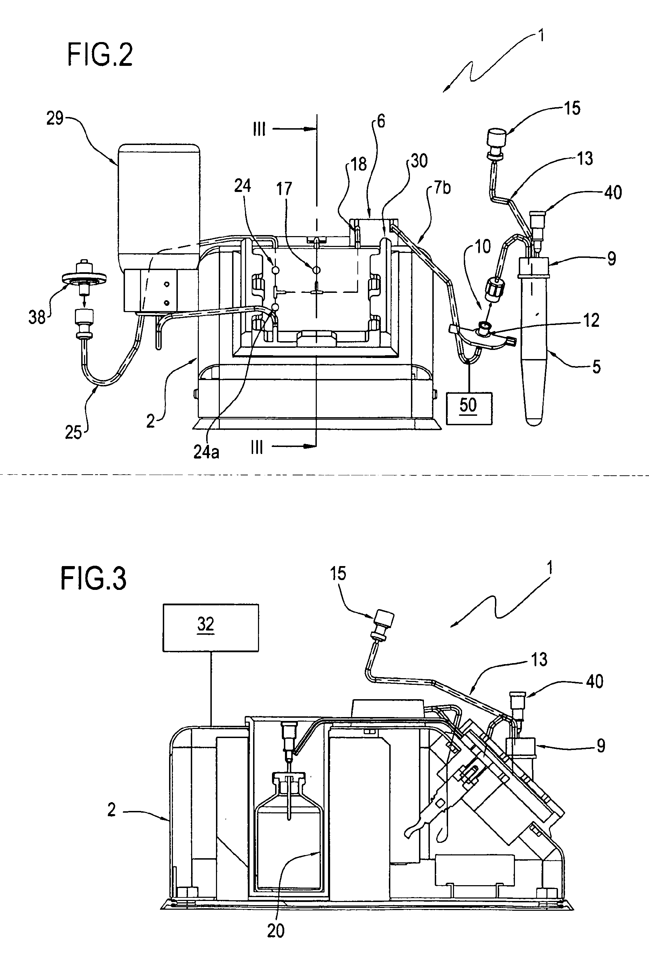

As illustrated in the accompanying drawings, and in particular with reference to Figures 1, 2 and 3, the numeral 1 denotes a dosing machine in accordance with the present invention.

In particular, the machine 1 is designed to dose a radioactive liquid substance or activity and comprises a main body 2 for supporting and containing a plurality of elements which are described in detail below.

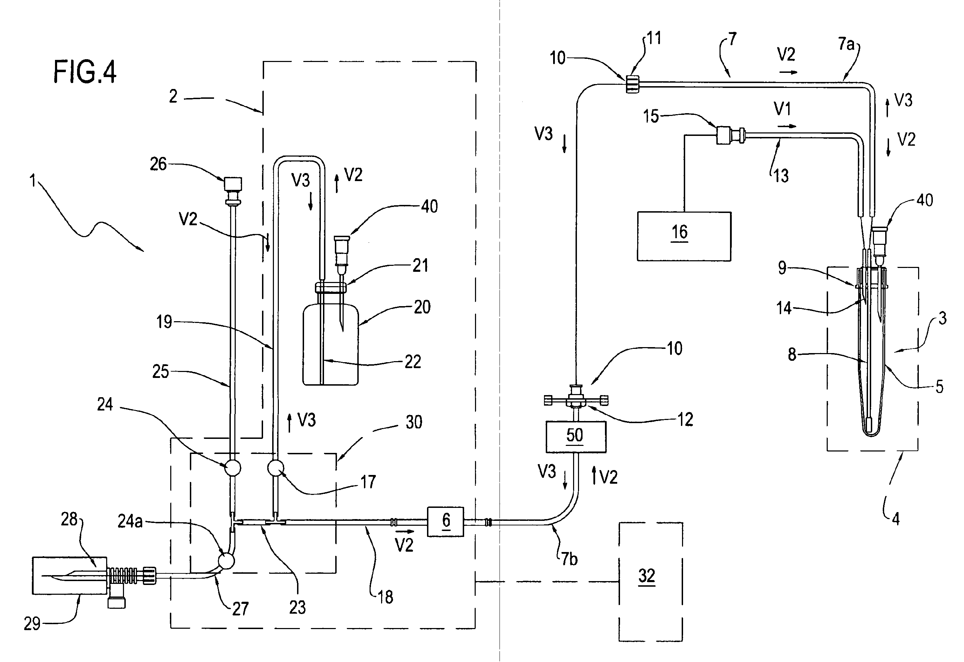

The machine 1 comprises a container 3 designed to be inserted in a dose calibrator 4, schematically illustrated with a dashed line in Figures 4, 5 and 7, of the substantially known type.

The dose calibrator 4 measures the total initial activity of the substance handled which is positioned in the calibrator 4 by means of the container 3 and the activity of each individual syringe or vial dispensed, as described in further detail below.

Advantageously, the dose calibrator 4, when not necessary for use with the machine 1 described herein, may be used for different activities, thus freeing up an additional resource for the operator.

In the preferred embodiment illustrated, the container 3 consists of an intermediate collecting vial 5.

The vial 5 is in fluid communication with a pump 6 attached to the main body 2.

In particular, the vial 5 is connected to the pump 6 by a first tube 7.

The tube 7 leads into the vial 5 by means of a cannula 8 inserted through a container 3 closing lid 9.

In the preferred embodiment illustrated, the tube 7 consists of a first portion 7a and a second portion 7b.

The first portion 7a is connected to the cannula 8, whilst the second portion 7b is connected to the pump 6.

The first and second portions 7a, 7b are connected to one another by suitable releasable connecting means 10.

The releasable connecting means 10 comprise first engagement means 11 attached to the portion 7a and second engagement means 12 attached to the portion 7b suitably prepared for connection to one another in such a way that the container 3 can substantially be separated from the pump 6, for uses described in more detail below.

A second tube 13 is in communication with the inside of the container 3 by means of a relative cannula 14.

The tube 13 also has relative engagement means 15 for connection to a synthesis module 16 for the radioactive liquid, of the substantially known type and therefore not described in detail.

As illustrated particularly in Figure 4, the pump 6 is in fluid communication with a second container 20 by means of a valve 17 and a pair of tubes 18, 19.

The container 20 is closed by a cap 21, preferably made of rubber, through which the tube 19 is in fluid communication with the inside of the container 20 by means of a suitable cannula 22.

As described in more detail below, the container 20 is used in the machine as a storage vial from which the radioactive substance is drawn, or to which the substance is returned at the end of the operating cycles.

The pump 6 is also in fluid communication by means of the tube 18, a pair of tubes 23, 25 and a second valve 24 with engagement means 26 for coupling to external accessories described in more detail below.

The means 26 form an inlet for air or for external substances.

The pump 6 is also in fluid communication by means of the tube 18, a tube 27 and a third valve 24a with perforator means 28, of the substantially known type, in particular designed to allow communication between the pump 6 and external containers.

The perforator means 28 are preferably designed to be inserted in a vial 29 of saline as described in detail below.

In the preferred embodiment illustrated, the valves 17, 24 and 24a are installed on the body 2 and operate on the relative tubes installed on a supporting element 30 which is attached to the body 2.

In particular, the element 30 is attached to the main body 2 in a releasable way by suitable coupling means 31 so that the set of tubes can easily be removed and substituted by means of the supporting element 30.

In a preferred embodiment the support 30 is obtained by moulding plastic material and is designed for "disposable" use, since it is easily applied and removed from the machine together with the relative tubes and connectors with a simple sliding joint 30a.

A computerised control unit, schematically illustrated with a block 32, monitors and saves the main data in the machine 1 and allows a generic user to intervene, influencing operation of the machine 1.

Figure 4 shows how in the configuration illustrated the machine 1 allows the container 20 which acts as a storage vial to be filled with a quantity of radioactive substance which has precisely defined physical characteristics and is suitably dosed.

In more detail, the vial 5 is connected to the synthesis module 16 by the tube 13 and the relative means 15 for engagement with the module 16.

When an operating cycle is started, the synthesis module 16 sends the radioactive substance to the vial 5, preferably already positioned in the dose calibrator 4, so that its total activity can be measured.

The radioactive substance flows, in a substantially known way, in the tube 13 in a direction V1, from the synthesis module 16 to the vial 5.

The total transfer of the substance from the synthesis module 16 to the vial 5 is preferably checked when the activity reading in the dose calibrator 4 stabilises, in accordance with a substantially known method.

If a previous cycle is to be maintained, the residual radioactive substance present in the storage vial 20 is automatically transferred to the collecting vial 5.

Driven by the pump 6, the residual radioactive substance flows in a direction V2 in the tubes 19, 18, 7 through the valve 17, appropriately opened, from the storage vial 20 to the collecting vial 5.

At this point the activity of the radioactive substance in the collecting vial 5 in the dose calibrator 4 is measured.

When the measurement is complete, the radioactive substance is transferred to the storage vial 20 by the pump 6 and through the valve 17, appropriately opened.

The radioactive substance flows, in a direction V3, along the tubes 7, 18, 19 from the collecting vial 5 to the storage vial 20.

In particular, the radioactive substance is preferably transferred in a controlled way, so that the total activity and volume arriving are known.

Completion of the transfer of the radioactive activity to the storage vial 20 is preferably checked when the reading of the residual activity in the collecting vial 5 stabilises.

It should be noticed that by inserting the perforator means 28 in the vial 29 of saline, by appropriately opening the valve 24a, it is possible to dilute the substance in the collecting vial 5.

In practice, the user decides the desired operating concentration and the machine 1 transfers the quantity of saline necessary to achieve that concentration.

At the end of the cycle, the above-mentioned control unit 32 updates the volume, activity and concentration of the radioactive substance now present in the storage vial 20.

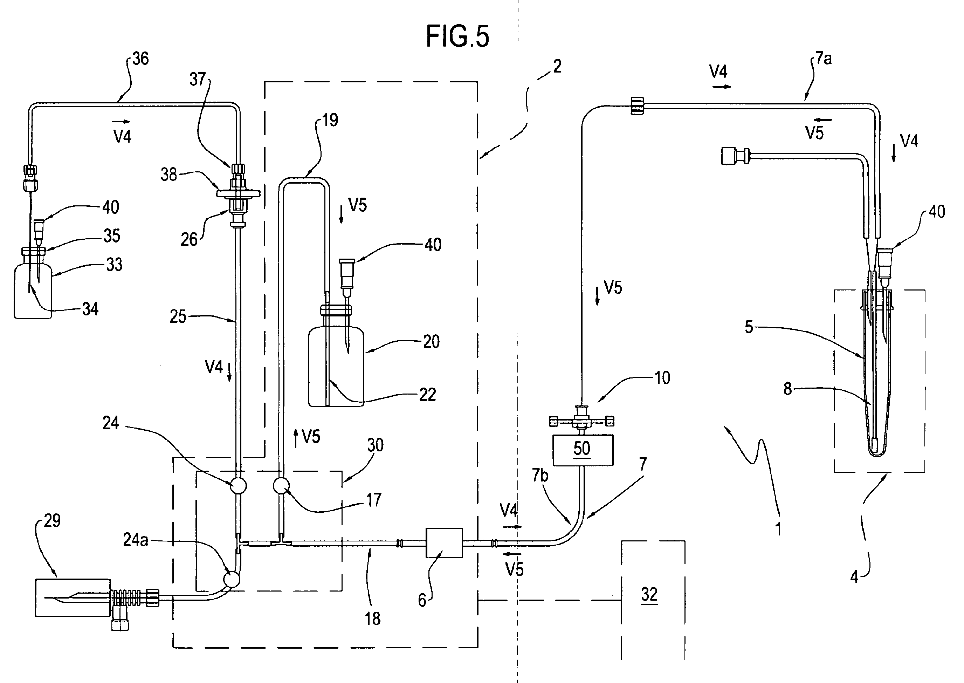

Figure 5 shows how in the configuration illustrated the machine 1 allows the container 20 which acts as a storage vial to be filled with a quantity of radioactive substance drawn from an external container or vial 33 acting as an external supply vial.

The external vial 33 is put in communication with the machine 1 tube 25 by a cannula 34 inserted in the vial 33 through a cap 35.

The cannula 34 preferably consists of a spinal needle of the substantially known type.

In particular, the cannula 34 is attached to a tube 36 which is connected to the engagement means 26.

In particular, the tube 36 has second engagement means 37 for releasable coupling with the engagement means 26.

Filtering means 38 of the substantially known type are preferably positioned between the first and second engagement means 26, 37.

The content of the external vial 33 is transferred, by the pump 6, to the collecting vial 5, preferably already positioned in the dose calibrator 4, through the valve 24, opened appropriately.

The radioactive substance present in the external vial 33 flows, in a direction V4, through the tubes 36, 25, 18, 7 to the collecting vial 5.

Once the total activity of the radioactive substance drawn from the external vial 33 has been measured in the collecting vial 5, the radioactive substance is transferred from the collecting vial 5 to the storage vial 20.

The radioactive substance flows, in a direction V5, from the collecting vial 5 to the storage vial 20 through the valve 17 and the tubes 7, 18 and 19.

Known volumes of radioactive substance are preferably transferred, so that the total activity and volume arriving in the storage vial 20 are known.

In a substantially similar way to that described above, through suitable adjustments to the valves 17 and 24a, the radioactive substance can be diluted with the saline.

The user decides the desired operating concentration and the computerised unit 32 transfers the quantity of saline necessary to achieve that concentration in the storage vial 20.

At the end of the process, the computerised unit 32 updates the volume, activity and concentration of the radioactive substance present in the storage vial 20.

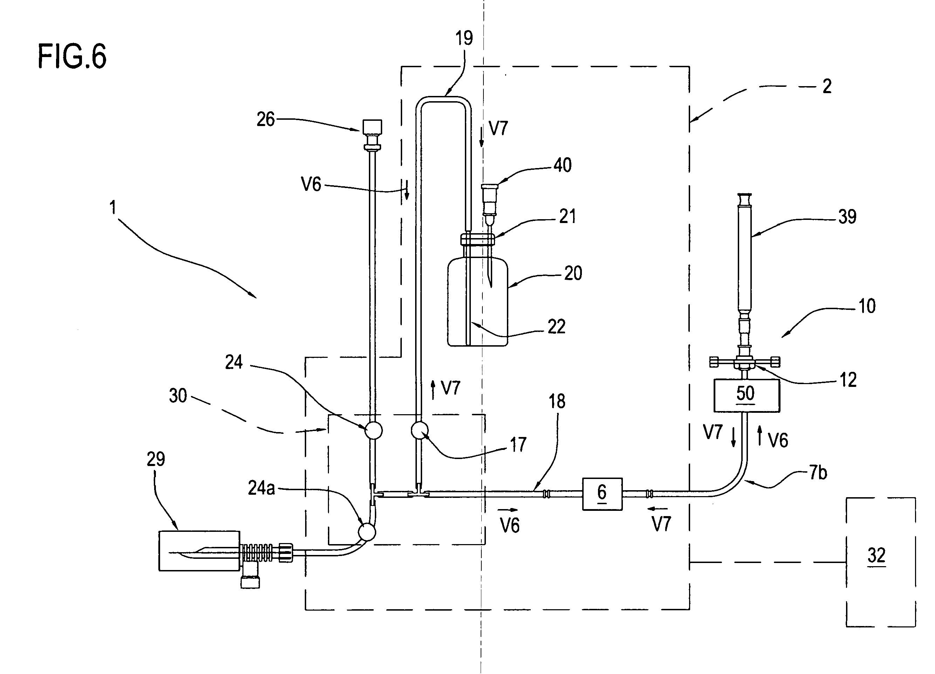

Figure 6 shows how, in the configuration illustrated, the machine 1 allows at least a syringe 39 to be filled.

In this configuration, the machine 1 is used for so-called "syringe fractionation".

It should be noticed that the tube 7 is divided into its two portions 7a and 7b by opening the connecting means 10.

The syringe 39, preferably shielded, engages with the portion 7b of the tube 7 at the second engagement means 12.

The computerised control unit 32 is used to enter in the machine 1 the data relating to the syringe 39 for a first patient and an operating cycle is initialised.

A microbolus of FDG, followed by saline, is sent into the shielded syringe 39 intended for the first patient.

In practice, the microbolus flows in a direction V6 from the storage vial 20 to the syringe 39, followed by the saline from the vial 29.

The remaining activity is then sent.

The substance for the syringes 39 for successive patients is fractionated by maintaining the separate boluses logic, until the end of the operating cycle. It should be noticed that at the end of each cycle, a bolus of saline is preferably sent from the vial 29 towards the tube 7b.

The bolus sent towards the tube 7b is then transferred, in a direction V7, from the tube 7b towards the storage vial 20 to keep the tubes 19, 18 forming a dispensing line clean.

The latter procedure prevents contamination of the machine 1 user during a change of configuration.

In particular, said procedure allows recovery of the end of cycle residues or the end of treatment waste without the use of any additional tubing or pumps, significantly simplifying the machine 1 compared with prior art machines.

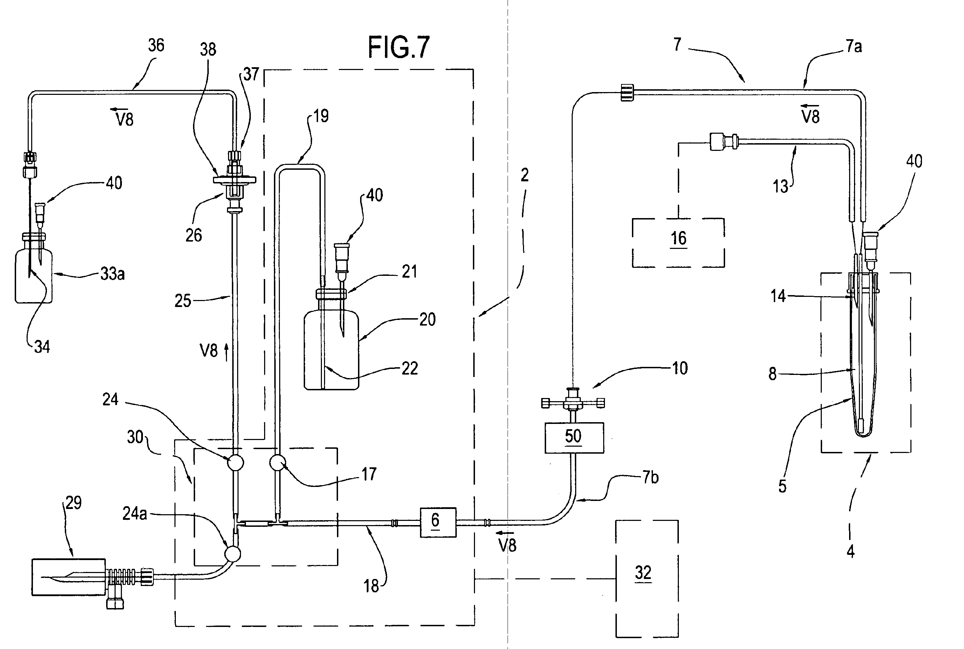

Figure 7 shows how, in the configuration illustrated, the machine 1 allows an external container or vial 33a for transfer of the radioactive substance to be filled.

The configuration illustrated in Figure 7 is used for "vial fractionation".

The computerised control unit 32 is used to enter in the machine 1, that is to say, in a machine control software, the data relating to the external vials 33a to be produced.

The transfer vial 33a is prepared by inserting into it the cannula or spinal needle 34 communicating with the valve 24.

When an operating cycle is initialised, a microbolus of FDG, from the storage vial 20, is sent to the collecting vial 5.

Once the required activity has been reached, measured by the calibrator 4, the machine 1 automatically transfers the content of the collecting vial 5 to the external transfer vial 33a. The radioactive substance flows in a direction V8 from the collecting vial 5 to the external vial 33a. The activity effectively transferred to the external vial 33a is, substantially, the result of the activity calibrated in the collecting vial 5 before the transfer, minus the residual activity present in the collecting vial 5 after the transfer.

The substance for the successive external vials 33a is fractionated by maintaining the separate boluses logic, until the end of the cycle.

At the end of the cycle, the machine 1 washes the tubes and the valves by running an automatic process for washing with saline.

The saline is drawn from the vial 29 and sent to the collecting vial 5, before being transferred to the storage vial 20.

It should be noticed that in the configurations described above the container 20 acting as a storage vial, the external vial 33, 33a and the collecting vial 5 each have, in the known way, a suitable breather needle 40.

The breather needle 40 is preferably of the filtered type.

The invention described brings important advantages. The dosing machine is a multi-purpose machine and in particular allows a substance to be fractionated into syringes and vials, measuring the activity with a single dose calibrator, external if necessary.

The machine is simple and versatile yet guarantees maximum measuring precision during all of the operating steps.

Fitting the set of tubes on the dedicated supporting element allows rapid set interchangeability, in particular for successive handling of different radioactive substances or different production batches of the same radioactive drug.

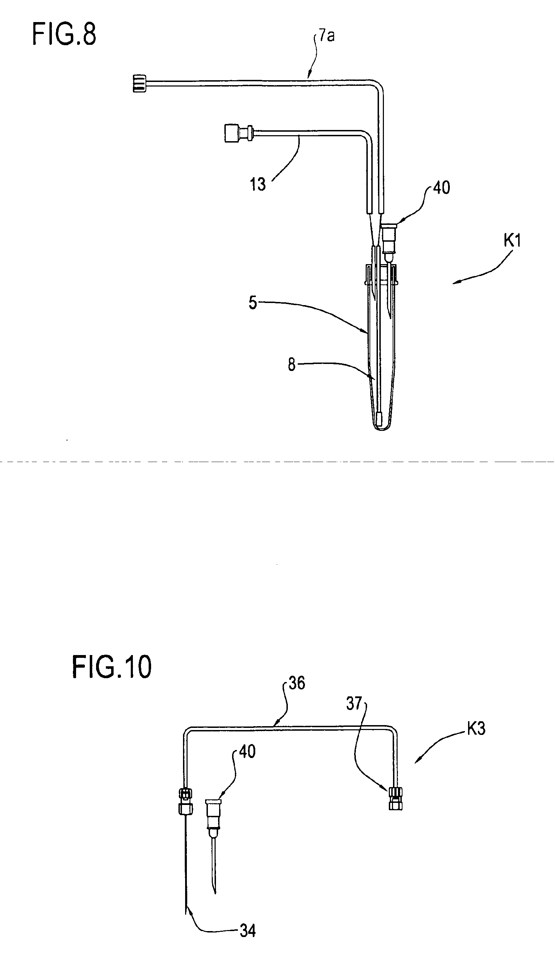

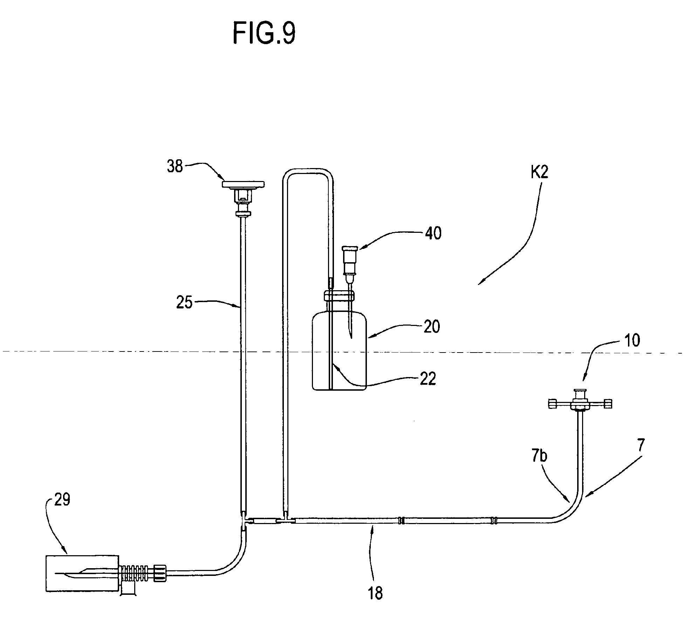

With reference to Figures 8 to 16, machine operation in various specific configurations is described, with corresponding kits of disposable components illustrated in the relative Figures 8 to 10.

In more detail, Figures 8 to 10 respectively illustrate three separate kits K1 K3, each consisting of components already described and labelled with the same reference characters.

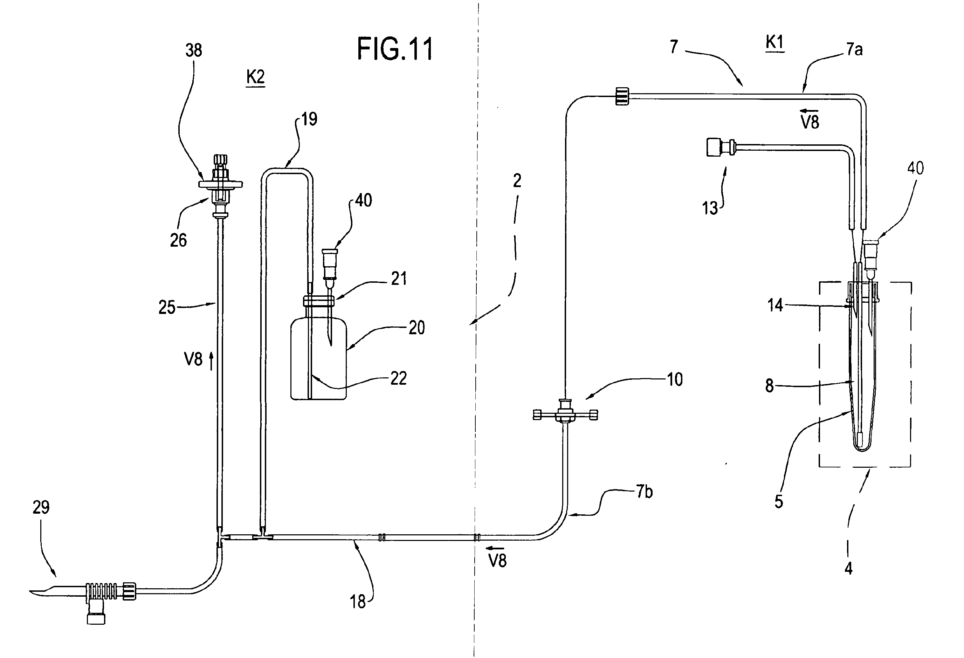

With reference to Figures 11 to 16, the following configurations for use may be seen:

Figure 11, kits K1 and K2 are used.

This configuration involves:
arrival of the radioactive liquid in the collecting vial 5 from the synthesis module, not illustrated, through the tube 13,
measurement of the activity with the calibrator 4, and
filling of storage vial 20 and diluting with saline from the vial 29 if necessary.

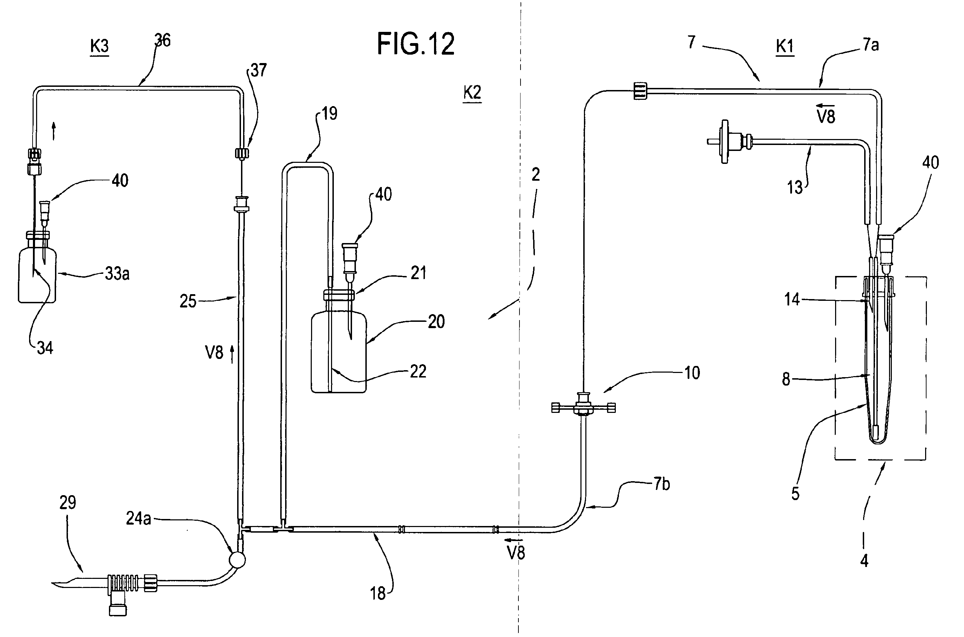

Figure 12. This configuration involves:
arrival of the radioactive liquid from the external vial 33a through the tube 36,
measurement of the activity with the calibrator 4 by transit of the liquid from the intermediate collecting vial 5, using the methods already described, and
filling of storage vial 20 and diluting with saline from the vial 29 if necessary.

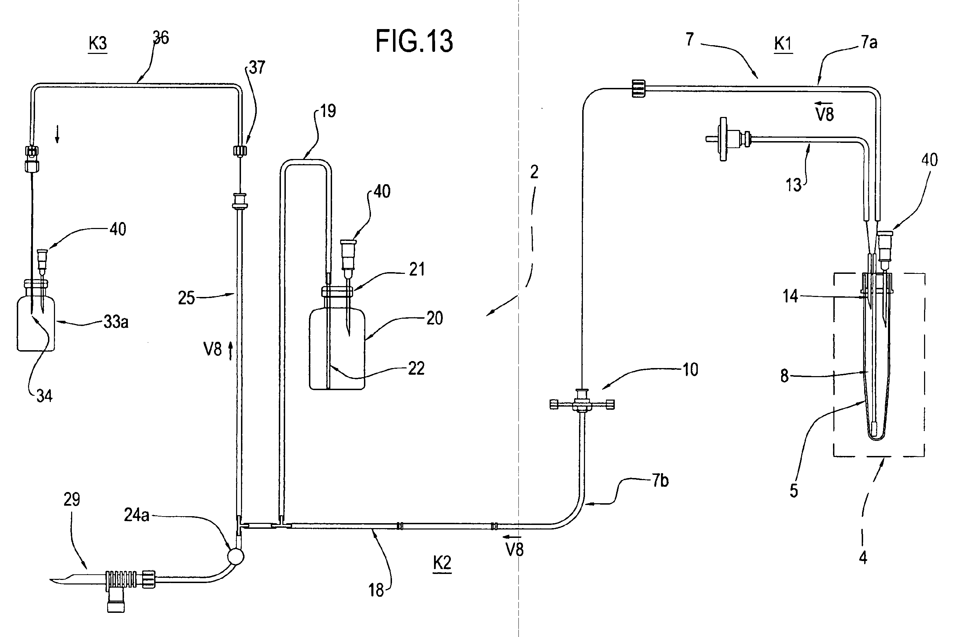

Figure 13, step after the configurations in Figures 11 and 12, kit K3 is added.

This configuration involves:
fractionating the activity from the previously filled storage vial 20 into external vials 33a to be delivered, and diluting with saline if necessary, and
reading the activity by transit of the liquid from the intermediate collecting vial 5.

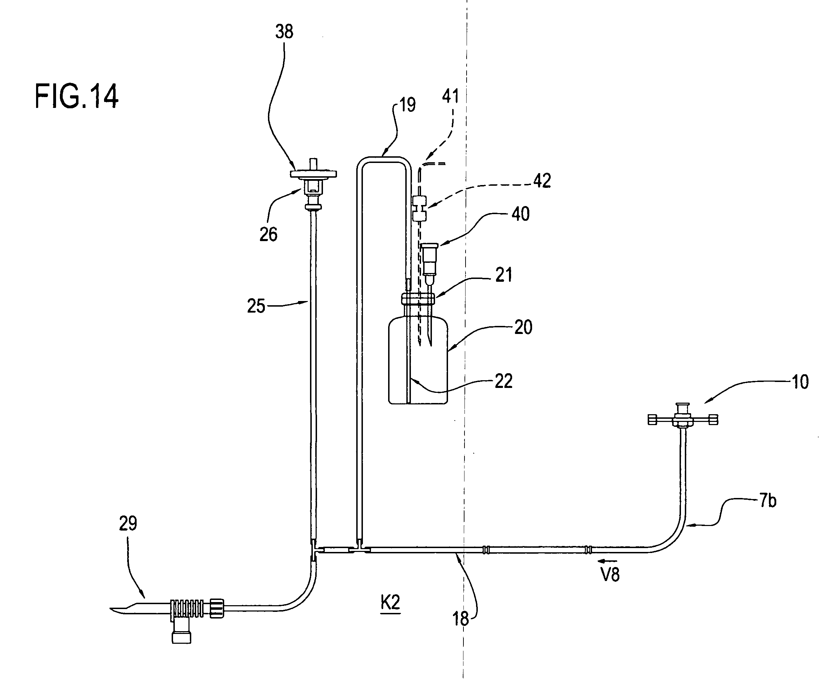

Figure 14, only kit K2 is used.

This configuration involves:
arrival of the radioactive liquid in the storage vial 20 from the synthesis module, not illustrated, through an external tube 41 with a needle 42 if necessary, and diluting with saline from the vial 29 if necessary.

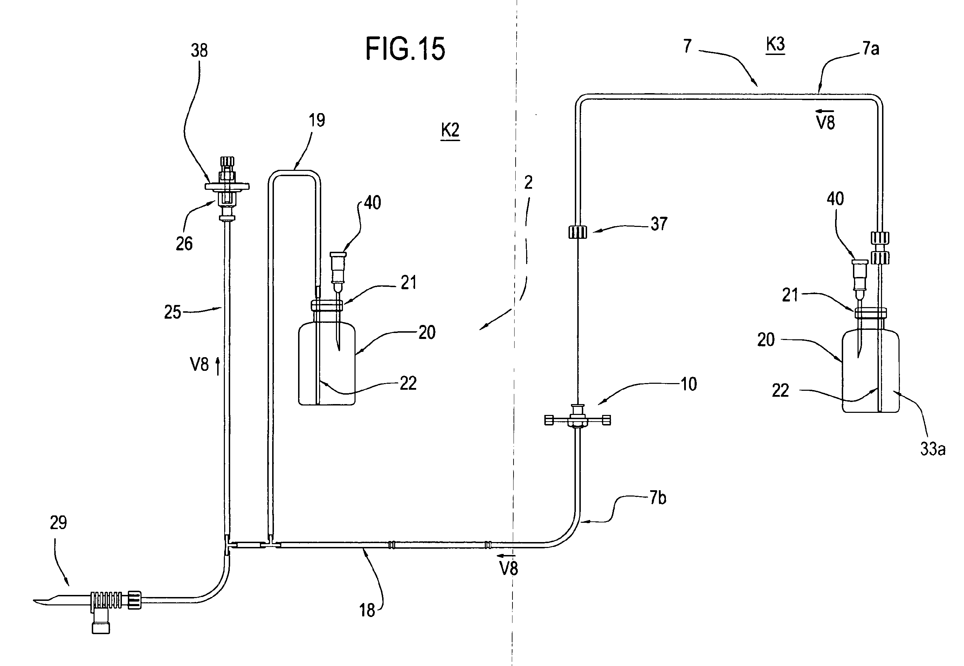

Figure 15, use of kits K2 and K3, connecting the joint 37 of kit K3 to the connector 10 of kit K2.

This configuration involves:
arrival of the radioactive liquid from the external vial 33a, and
filling of storage vial 20 and diluting with saline from the vial 29 if necessary.

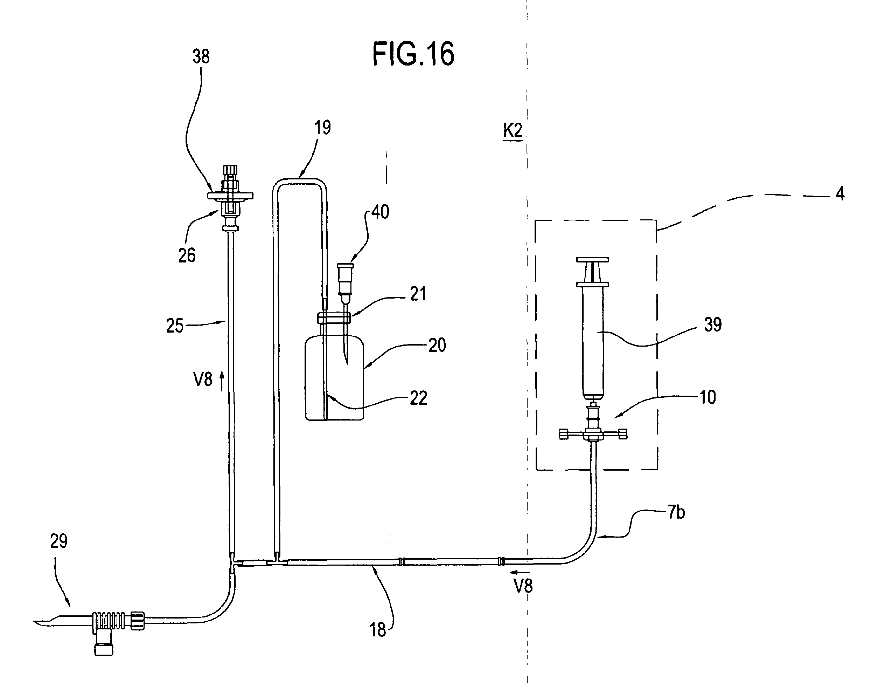

Figure 16, is the "end of line" configuration which comes after all of the configurations already described. This only uses kit K2, with a storage vial 20 filled with liquid to be fractionated.

This configuration involves:
drawing the radioactive liquid already fed into the storage vial 20, and
filling of syringes 39 by fractionating the liquid in the storage vial 20, and diluting with saline from the vial 29 if necessary, and
measurement of the activity in the syringe inserted in the calibrator 4.

The invention described has evident industrial applications and can be modified and adapted without thereby departing from the scope of the inventive concept. Moreover, all details of the invention may be substituted by technically equivalent elements.

## Claims

1. A dosing machine for radioactive liquid substances comprising a separate body (2) supporting means for fractionating a quantity of radioactive liquid substance, comprising:
an inlet (22) for the radioactive liquid substance to be fractionated,
an inlet (28) for a second liquid substance,
a connector (26) for letting in air,
a single reversible peristaltic pump (6) for fluid transfer,
first tubing (19, 18) connecting the inlet (22) for the radioactive liquid substance to the reversible pump (6),
second tubing (25, 23, 18) connecting the air inlet to the reversible pump (6),
third tubing (27, 23, 18) connecting the saline inlet (28) to the reversible pump (6),
a first valve (17) positioned between the inlet (22) for the radioactive liquid substance and the reversible pump (6),
a second valve (24) positioned between the air inlet and the reversible pump (6),
a third valve (24a) positioned between the saline inlet (28) and the reversible pump (6),
an air filter (38) which can be removably attached to the connector (26),
a tube (7b) for connecting the reversible pump (6) to a vial or syringe (39) for collecting the radioactive liquid substance transferred by the pump.

2. The machine according to claim 1, **characterised in that** it comprises means for receiving a radioactive liquid, means for sending a quantity of liquid to a dose calibrator (4) and means for feeding a calibrated dose of liquid into a vial or syringe (20, 33a, 39), means for transferring a quantity of liquid to an intermediate collecting vial (5) which can be inserted in the dose calibrator (4) and means for transferring at least part of the quantity of liquid from the intermediate collecting vial (5) to the means for feeding a calibrated dose of liquid into a vial or syringe (20, 33a, 39).

3. The machine according to claim 2, **characterised in that** the means for receiving a radioactive liquid and the means for sending a quantity of liquid to the dose calibrator (4) comprise a connection to an external synthesis module (16).

4. The machine according to claim 3, **characterised in that** the means for feeding a calibrated dose of liquid into a vial or syringe (20, 33a, 39) comprise carrying and pumping means (19, 17, 18, 6, 7b) in fluid communication with a storage vial (20).

5. The machine according to claim 4, **characterised in that** the means for transferring at least part of the quantity of liquid from the intermediate collecting vial (5) to the means for feeding a calibrated dose of liquid into the storage vial (20) comprise a first tube (7a) in communication with the intermediate collecting vial (5) by means of a respective cannula (8).

6. The machine according to claim 2, **characterised in that** the means for receiving a radioactive liquid comprise an external supply vial (33), the means for sending a quantity of liquid to the dose calibrator comprising carrying and pumping means (36, 25, 24, 18, 6, 7b), operating between the external supply vial (33) and the dose calibrator (4).

7. The machine according to claim 6, **characterised in that** the means for feeding a calibrated dose of liquid into a vial or syringe (20) comprise second carrying and pumping means (7b, 6, 18, 17, 19), operating between the intermediate collecting vial (5) and a storage vial (20).

8. The machine according to claim 7, **characterised in that** the means for transferring at least part of the quantity of liquid from the intermediate collecting vial (5) to the means for feeding a calibrated dose of liquid into a vial or syringe comprise a first tube (7a) in communication with the intermediate collecting vial (5) by means of a respective cannula (8).

9. The machine according to claim 2, **characterised in that** the means for feeding a calibrated dose of liquid into a vial or syringe comprise carrying and pumping means (34, 36, 25, 24, 18, 6, 7b) in fluid communication with an external vial (33a).

10. The machine according to claim 9, **characterised in that** the means for receiving a radioactive liquid comprise a storage vial (20).

11. The machine according to claim 10, **characterised in that** the means for sending a quantity of liquid to the dose calibrator comprise second carrying and pumping means (22, 19, 17, 18, 6, 7b).

12. The machine according to claim 11, **characterised in that** the means for transferring the quantity of liquid to an intermediate collecting vial (5) which can be inserted in the dose calibrator (4) comprise carrying means (7a, 8) which lead into the intermediate collecting vial (5).

13. The machine according to claim 2, **characterised in that** the means for feeding a calibrated dose of liquid into a vial or syringe (20, 33a, 39) comprise carrying and pumping means (22, 19, 17, 18, 6, 7b) which lead into a shielded syringe (39).

## Patentansprüche

1. Dosiergerät für radioaktive, flüssige Substanzen mit einem separaten Körper (2), der Einrichtungen zum Fraktionieren einer Menge radioaktiver, flüssiger Substanz trägt, Folgendes umfassend:
einen Einlass (22) für die zu fraktionierende radioaktive, flüssige Substanz,
einen Einlass (28) für eine zweite flüssige Substanz,
ein Anschlussteil (26) zum Einlassen von Luft,
eine einzige umsteuerbare Peristaltik-Pumpe (6) zum Fluidtransport,
erstes Schlauchmaterial (19, 18), das den Einlass (22) für die radioaktive, flüssige Substanz mit der umsteuerbaren Pumpe (6) verbindet,
zweites Schlauchmaterial (25, 23, 18), das den Lufteinlass mit der umsteuerbaren Pumpe (6) verbindet,
drittes Schlauchmaterial (27, 23, 18), das den Einlass (28) für physiologische Kochsalzlösung mit der umsteuerbaren Pumpe (6) verbindet,
ein erstes Ventil (17), das zwischen dem Einlass (22) für die radioaktive, flüssige Substanz und der umsteuerbaren Pumpe (6) positioniert ist,
ein zweites Ventil (24), das zwischen dem Lufteinlass und der umsteuerbaren Pumpe (6) positioniert ist,
ein drittes Ventil (24a), das zwischen dem Einlass (28) für physiologische Kochsalzlösung und der umsteuerbaren Pumpe (6) positioniert ist,
einen Luftfilter (38), der austauschbar an dem Anschlussteil (26) befestigt sein kann,
einen Schlauch (7b) zum Verbinden der umsteuerbaren Pumpe (6) mit einem Fläschchen oder einer Spritze (39) zum Aufnehmen der mit Hilfe der Pumpe transportierten radioaktiven, flüssigen Substanz.

2. Gerät nach Anspruch 1, **dadurch gekennzeichnet, dass** es eine Einrichtung zum Entgegennehmen einer radioaktiven Flüssigkeit, eine Einrichtung zum Schicken einer Menge Flüssigkeit an einen Dosiskalibrator (4) und eine Einrichtung zum Einfüllen einer kalibrierten Dosis Flüssigkeit in ein Fläschchen oder eine Spritze (20, 33a, 39), eine Einrichtung zum Transportieren einer Menge Flüssigkeit zu einem Zwischenprodukt-Sammelfläschchen (5), das in den Dosiskalibrator (4) eingebracht werden kann, und eine Einrichtung zum Transportieren mindestens eines Teils der Menge Flüssigkeit aus dem Zwischenprodukt-Sammelfläschchen (5) zu der Einrichtung zum Einfüllen einer kalibrierten Dosis Flüssigkeit in ein Fläschchen oder eine Spritze (20, 33a, 39) umfasst.

3. Gerät nach Anspruch 2, **dadurch gekennzeichnet, dass** die Einrichtung zum Entgegennehmen einer radioaktiven Flüssigkeit und die Einrichtung zum Schicken einer Menge Flüssigkeit an den Dosiskalibrator (4) eine Verbindung zu einem externen Synthesemodul (16) umfassen.

4. Gerät nach Anspruch 3, **dadurch gekennzeichnet, dass** die Einrichtung zum Einfüllen einer kalibrierten Dosis Flüssigkeit in ein Fläschchen oder eine Spritze (20, 33a, 39) eine Beförderungs- und Pumpeinrichtung (19, 17, 18, 6, 7b) in Fluidverbindung mit einem Aufbewahrungsfläschchen (20) umfasst.

5. Gerät nach Anspruch 4, **dadurch gekennzeichnet, dass** die Einrichtung zum Transportieren mindestens eines Teils der Menge Flüssigkeit aus dem Zwischenprodukt-Sammelfläschchen (5) zu der Einrichtung zum Einfüllen einer kalibrierten Dosis Flüssigkeit in das Aufbewahrungsfläschchen (20) einen ersten Schlauch (7a) in Verbindung mit dem Zwischenprodukt-Sammelfläschchen (5) über eine zugehörige Kanüle (8) umfasst.

6. Gerät nach Anspruch 2, **dadurch gekennzeichnet, dass** die Einrichtung zum Entgegennehmen einer radioaktiven Flüssigkeit ein externes Vorratsfläschchen (33) umfasst, wobei die Einrichtung zum Schicken einer Menge Flüssigkeit an den Dosiskalibrator eine Beförderungs- und Pumpeinrichtung (36, 25, 24, 18, 6, 7b) umfasst, die zwischen dem externen Vorratsfläschchen (33) und dem Dosiskalibrator (4) wirksam ist.

7. Gerät nach Anspruch 6, **dadurch gekennzeichnet, dass** die Einrichtung zum Einfüllen einer kalibrierten Dosis Flüssigkeit in ein Fläschchen oder eine Spritze (20) eine zweite Beförderungs- und Pumpeinrichtung (7b, 6, 18, 17, 19) umfasst, die zwischen dem Zwischenprodukt-Sammelfläschchen (5) und einem Aufbewahrungsfläschchen (20) wirksam ist.

8. Gerät nach Anspruch 7, **dadurch gekennzeichnet, dass** die Einrichtung zum Transportieren mindestens eines Teils der Menge Flüssigkeit aus dem Zwischenprodukt-Sammelfläschchen (5) zu der Einrichtung zum Einfüllen einer kalibrierten Dosis Flüssigkeit in ein Fläschchen oder eine Spritze einen ersten Schlauch (7a) in Verbindung mit dem Zwischenprodukt-Sammelfläschchen (5) über eine zugehörige Kanüle (8) umfasst.

9. Gerät nach Anspruch 2, **dadurch gekennzeichnet, dass** die Einrichtung zum Einfüllen einer kalibrierten Dosis Flüssigkeit in ein Fläschchen oder eine Spritze eine Beförderungs- und Pumpeinrichtung (34, 36, 25, 24, 18, 6, 7b) in Fluidverbindung mit einem externen Fläschchen (33a) umfasst.

10. Gerät nach Anspruch 9, **dadurch gekennzeichnet, dass** die Einrichtung zum Entgegennehmen einer radioaktiven Flüssigkeit ein Aufbewahrungsfläschchen (20) umfasst.

11. Gerät nach Anspruch 10, **dadurch gekennzeichnet, dass** die Einrichtung zum Schicken einer Menge Flüssigkeit an den Dosiskalibrator eine zweite Beförderungs- und Pumpeinrichtung (22, 19, 17, 18, 6, 7b) umfasst.

12. Gerät nach Anspruch 11, **dadurch gekennzeichnet, dass** die Einrichtung zum Transportieren der Menge Flüssigkeit zu einem Zwischenprodukt-Sammelfläschchen (5), das in den Dosiskalibrator (4) eingebracht werden kann, Beförderungsmittel (7a, 8) umfasst, die in das Zwischenprodukt-Sammelfläschchen (5) leiten.

13. Gerät nach Anspruch 2, **dadurch gekennzeichnet, dass** die Einrichtung zum Einfüllen einer kalibrierten Dosis Flüssigkeit in ein Fläschchen oder eine Spritze (20, 33a, 39) eine Beförderungs- und Pumpeinrichtung (22, 19, 17, 18, 6, 7b) umfasst, die in eine abgeschirmte Spritze (39) leitet.

## Revendications

1. Une machine de dosage pour substances liquides radioactives comprenant un corps (2) distinct supportant des moyens pour fractionner une quantité de substance liquide radioactive, comprenant :
une entrée (22) pour la substance liquide radioactive à fractionner,
une entrée (28) pour une deuxième substance liquide,
un raccord (26) pour l'entrée d'air,
une seule pompe réversible (6), péristaltique, pour le transfert de fluide,
une première tubulure (19, 18) raccordant l'entrée (22) pour la substance liquide radioactive à la pompe réversible (6),
une deuxième tubulure (25, 23, 18) raccordant l'entrée d'air à la pompe réversible (6),
une troisième tubulure (27, 23, 18) raccordant l'entrée (28) de solution saline à la pompe réversible (6),
une première soupape (17) placée entre l'entrée (22) pour la substance liquide radioactive et la pompe réversible (6),
une deuxième soupape (24) placée entre l'entrée d'air et la pompe réversible (6),
une troisième soupape (24a) placée entre l'entrée (28) de solution saline et la pompe réversible (6),
un filtre à air (38) qui peut être associé de façon amovible au raccord (26),
un tuyau (7b) pour raccorder la pompe réversible (6) à une fiole ou seringue (39) de collecte de la substance liquide radioactive transférée par la pompe.

2. La machine selon la revendication 1, **caractérisée en ce qu'**elle comprend des moyens pour recevoir un liquide radioactif, des moyens pour envoyer une quantité de liquide à un calibreur de doses (4) et des moyens pour alimenter une dose calibrée de liquide dans une fiole ou seringue (20, 33a, 39), des moyens pour transférer une quantité de liquide dans une fiole de collecte intermédiaire (5) qui peut être introduite dans le calibreur de doses (4), et des moyens pour transférer au moins une partie de la quantité de liquide de la fiole de collecte intermédiaire (5) à des moyens pour alimenter une dose calibrée de liquide dans une fiole ou seringue (20, 33a, 39).

3. La machine selon la revendication 2, **caractérisée en ce que** les moyens pour recevoir un liquide radioactif et les moyens pour envoyer une quantité de liquide au calibreur de doses (4) comprennent un raccord à un module de synthèse externe (16).

4. La machine selon la revendication 3, **caractérisée en ce que** les moyens pour alimenter une dose calibrée de liquide dans une fiole ou seringue (20, 33a, 39) comprennent des moyens d'amenée et de pompage (19, 17, 18, 6, 7b) en communication de fluide avec une fiole de stockage (20).

5. La machine selon la revendication 4, **caractérisée en ce que** les moyens pour transférer au moins une partie de la quantité de liquide de la fiole de collecte intermédiaire (5) aux moyens pour alimenter une dose calibrée de liquide dans la fiole de stockage (20) comprennent un premier tuyau (7a) en communication avec ladite fiole de collecte intermédiaire (5) par le biais d'une canule (8) respective.

6. La machine selon la revendication 2, **caractérisée en ce que** les moyens pour recevoir un liquide radioactif comprennent une fiole d'alimentation extérieure (33), les moyens pour envoyer une quantité de liquide au calibreur de doses comprenant des moyens d'amenée et de pompage (36, 25, 24, 18, 6, 7b) opérant entre la fiole d'alimentation extérieure (33) et le calibreur de doses (4).

7. La machine selon la revendication 6, **caractérisée en ce que** les moyens pour alimenter une dose calibrée de liquide dans une fiole ou seringue (20) comprennent des deuxièmes moyens d'amenée et de pompage (7b, 6, 18, 17, 19) opérant entre la fiole de collecte intermédiaire (5) et une fiole de stockage (20).

8. La machine selon la revendication 7, **caractérisée en ce que** les moyens pour transférer au moins une partie de la quantité de liquide de la fiole de collecte intermédiaire (5) aux moyens pour alimenter une dose calibrée de liquide dans une fiole ou seringue comprennent un premier tuyau (7a) en communication avec ladite fiole de collecte intermédiaire (5) par le biais d'une canule (8) respective.

9. La machine selon la revendication 2, **caractérisée en ce que** les moyens pour alimenter une dose calibrée de liquide dans une fiole ou seringue comprennent des moyens d'amenée et de pompage (34, 36, 25, 24, 18, 6, 7b) en communication de fluide avec une fiole extérieure (33a).

10. La machine selon la revendication 9, **caractérisée en ce que** les moyens pour recevoir un liquide radioactif comprennent une fiole de stockage (20).

11. La machine selon la revendication 10, **caractérisée en ce que** les moyens pour envoyer une quantité de liquide au calibreur de doses comprennent des deuxièmes moyens d'amenée et de pompage (22, 19, 17, 18, 6, 7b).

12. La machine selon la revendication 11, **caractérisée en ce que** les moyens pour transférer la quantité de liquide à une fiole de collecte intermédiaire (5) qui peut être introduite dans le calibreur de doses (4) comprennent des moyens d'amenée (7a, 8) qui débouchent dans ladite fiole de collecte intermédiaire (5).

13. La machine selon la revendication 2, **caractérisée en ce que** les moyens pour alimenter une dose calibrée de liquide dans une fiole ou seringue (20, 33a, 39) comprennent des moyens d'amenée et de pompage (22, 19, 17, 18, 6, 7b) qui débouchent dans une seringue blindée (39).
